(19) Europäisches Patentamt
European Patent Office
Office européen des brevets

(11) Veröffentlichungsnummer: 0 510 620 A1

# (12) EUROPÄISCHE PATENTANMELDUNG

(21) Anmeldenummer: **92106895.3**

(51) Int. Cl.[5]: **C07H 15/203**, C12Q 1/40

(22) Anmeldetag: **22.04.92**

(30) Priorität: **23.04.91 DE 4113238**

(43) Veröffentlichungstag der Anmeldung:
**28.10.92 Patentblatt 92/44**

(84) Benannte Vertragsstaaten:
**PT**

(71) Anmelder: **BOEHRINGER MANNHEIM GMBH**
**Sandhofer Strasse 112-132**
**W-6800 Mannheim-Waldhof(DE)**

(72) Erfinder: **Junius-Comer, Martina, Dr.**
**Am Bodenbach 5**
**W-8127 Iffeldorf(DE)**
Erfinder: **Schmidt, Axel, Dr.**
**Gudrunstrasse 18**
**W-8000 München 19(DE)**
Erfinder: **Rauscher, Elli, Dr.**
**Guardinistrasse 71**
**W-8000 München 70(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Kopernikusstrasse 9 Postfach 86 08**
**20**
**W-8000 München 86(DE)**

## (54) Indophenol-substituierte Maltooligoside als alpha-Amylase-Substrate.

(57) Die Erfindung betrifft neue indophenylsubstituierte Maltosederivate, deren Verwendung zur Bestimmung der enzymatischen Aktivität von α-Amylase und Reagenzien zur α-Amylase-Bestimmung, welche die neuen indophenylinduzierten Maltosederivate als Enzymsubstrat enthalten.

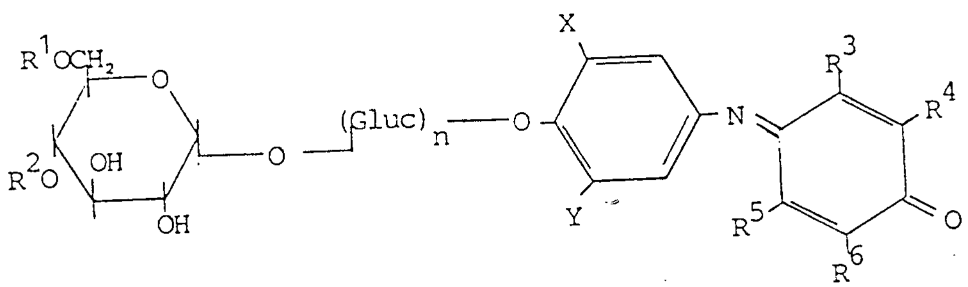

(I)

worin
Gluc ein Glucosemolekül ist und der Indophenolrest in α-Stellung mit dem Zucker verknüpft ist,
n = 1 oder 2,
X, Y = unabhänig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppen,
unter der Voraussetzung, daß X und Y nicht gleichzeitig H sind.

EP 0 510 620 A1

Fig. 1

2',2,6-Trichlorindophenyl-
alpha-maltotriosid ;

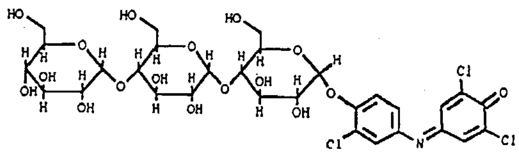

2,6-Dichlorindophenyl-
alpha-maltotriosid:

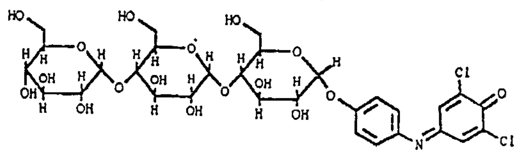

2,6-Dimethylindophenyl-
alpha-maltotriosid:

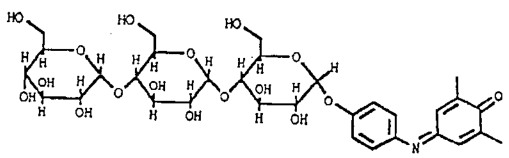

Die Erfindung betrifft Maltoside und Maltotrioside, die an ihrem reduzierenden Ende in α-Stellung mit einem substituierten Indophenolrest verknüpft sind und die Verwendung dieser Verbindungen als synthetische Enzymsubstrate zur direkten Bestimmung von α-Amylase, insbesondere zur Bestimmung von Pankreas-α-Amylase sowie ein dazu geeignetes Reagenz.

α-Amylase (E.C. 3.2.1.1) baut 1,4-α-glucosidisch verknüpfte Oligo- und Polysaccharide überwiegend durch Hydrolyse der 1,4-α-glucosidischen Bindungen zu Maltose und Maltooligosiden ab. Das Enzym hat neben der Verwendung bei der industriellen Fermentationstechnik erhebliche Bedeutung im Rahmen der klinischen Analytik und Diagnostik. Insbesondere hat die Bestimmung der Pankreas-α-Amylase erhebliche diagnostische Bedeutung, da bei zahlreichen Erkrankungen sich der Gehalt dieses Enzyms in Körperflüssigkeiten wie etwa Serum, Harn oder Duodenalsekret beträchtlich verändert.

Es sind eine Vielzahl von Verfahren zur Bestimmung der α-Amylase sowie dafür geeignete synthetische Enzymsubstrate bekannt.

Die EP-A 0346 912 offenbart Maltooligoside, die an ihrem reduzierenden Ende in α- oder β-Stellung mit einem Phenylrest verknüpft sind, der in Para-Position durch eine Nitro- oder Nitrovinylgruppe substituiert ist. Diese Maltooligoside werden als geeignete Substrate zur Bestimmung der Aktivität von Hydrolasen, Lipasen, Esterasen, α-Glucosidase oder Glucoamylase beschrieben.

Die EP-A 0 319 933 offenbart Maltooligoside, deren nicht reduzierendes Ende durch Schutzgruppen blockiert ist und die am reduzierenden Ende in α- oder β-Stellung mit substituierten oder nicht substituierten Phenylresten verknüpft sind. Beispiele für substituierte Phenylreste sind Halogen-, Hydroxyl-, Alkyl-, Alkoxy-, Alkoxycarbonyl-, Nitro- und Phenolsubstituierte Phenylreste. Besonders bevorzugt ist die Phenylrest durch eine oder mehrere Nitrogruppen substituiert. β-verknüpfte Maltooligoside des obigen Typs werden als geeignet zur Bestimmung von α-Amylase in Gegenwart von Hilfsenzymen wie etwa α- und β-Glucosidase bezeichnet. Eine direkte Bestimmung von α-Amylase ohne die Anwesenheit von Hilfsenzymen ist nicht offenbart.

Die japanische Offenlegungsschrift 64-42497 offenbart Maltooligoside, die an ihrem reduzierenden Ende in β-Stellung mit einem Indophenolrest der Formel

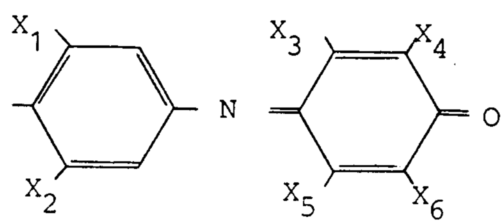

substituiert sind, wobei $X^1$ bis $X^6$ Wasserstoff- bzw. Halogen-Atome bzw. Nitro-, Cyan-, Azid-, Acyl-, Sulfonsäure-, Nitroso-, Sulfonyl-, Sulfoxyl-, Thiocyan-, Isothiocyan-, Isonitril-, Imino-, Azo-, Diazo-, Alkyl-, Allyl- bzw. Arylgruppen bedeuten, wobei $X^3$ und $X^4$ und/oder $X^6$ und $X^6$ verbunden sein und kondensierte aromatische Ringe bilden können.

Weiterhin werden Verfahren zur Herstellung dieser Verbindungen offenbart, sowie die Verwendung als α-Amylasesubstrate in Gegenwart von α- und β-Glucosidase. In den Beispielen werden konkret die Herstellung der Verbindungen Phenolindo-3'-chlorphenyl-β-maltopentaosid, Hexadecaacetyl-(phenolindo-3',5'-dichlorphenyl)-β-maltopentaosid, Phenolindo-3',5'-dichlorphenyl-β-maltoheptaosid, Hexadecaacetyl-(2,5-dimethylphenolindo-3'-chlorphenyl-β)-pentaosid und Hexadecaacetyl-(1-naphtholindo-3'-chlorphenyl)-β-pentaosid offenbart. Die EP-A 0 263 435 offenbart aromatisch substituierte Glycoside der Formel

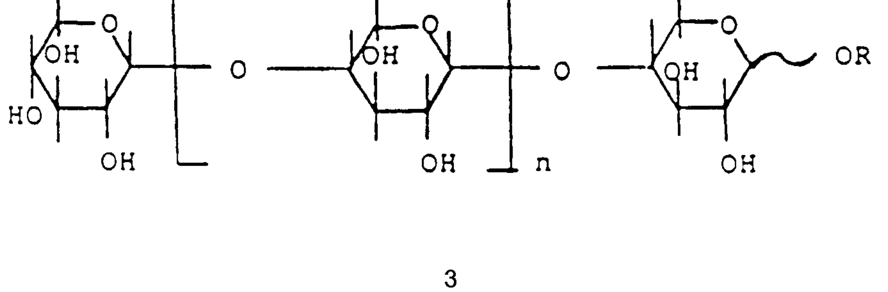

(I)

worin der Substituent R am reduzierenden Ende der Verbindung in α-Stellung vorliegt, n = 0 oder 1 ist und R ein substituierter aromatischer Rest ist, ausgewählt aus der Gruppe

(a) 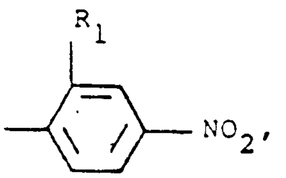 , (b) 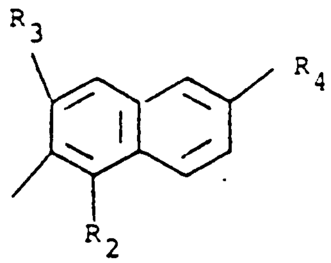 und (c) 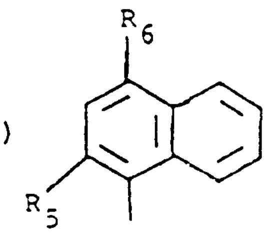

worin $R^1$ bis einschließlich $R^6$ unabhängig Halogen, $NO_2$, $SO_3H$,

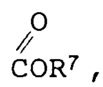 , 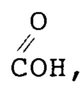 , 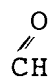 oder 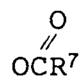

sind, wobei $R^7$ ein niederer Alkylrest ist, einschließlich seiner Stereoisomere, optischen Isomere und geometrischen Isomere und Mischungen dieser Isomere. Diese Substrate sind als direkte Substrate (d.h. ohne Anwesenheit von Hilfsenzymen) für α-Amylasen beschrieben. In den Beispielen wird konkret die Herstellung von 2-Chlor-4-nitrophenyl-α-D-maltotriosid, 4-Chlor-2-nitro-1-naphthyl-α-maltotriosid und 2-Formyl-4-nitrophenyl-α-D-maltotriosid offenbart. Weiterhin wird ein Verfahren für die Herstellung der Substrate und verwandter Substanzen offenbart. Die in der EP-A 0 263 435 offenbarten Verbindungen weisen eine maximale Absorption bei Wellenlängen von ca. 385 bis 450 nm auf.

Die japanische Offenlegungsschrift 02/306990 beschreibt Indophenyl-α-glycoside mit 2 bis 10 Glucoseeinheiten, Indophenylresten als Chromophor und unsubstituierten OH-Gruppen am Oligoglucosid. Als Beispiel offenbart ist Phenolindo-3'-chlorphenyl-α-D-maltopentaosid, dessen Bestimmung in Gegenwart des Hilfsenzyms α-Glucosidase erfolgt.

Die japanische Offenlegungsschrift 02/306991 offenbart cyclische Acetale von Indophenylglucosiden mit α- oder β-Verknüpfungund 2 bis 10 Glucoseeinheiten, die Indophenylreste als Chromophor aufweisen und bei denen die OH-Gruppen in den Positionen $C_4$ und $C_6$ der Glucose am nicht reduzierenden Ende durch Alkyl- oder Alkylidenreste substituiert sind. Als bevorzugt werden Verbindungen mit 5 bis 7 Glucoseeinheiten bezeichnet. Als Beispiel ist Isopropylidenphenolindophenyl-α-maltopentaosid offenbart, dessen Bestimmung in Gegenwart der Hilfsenzyme α- und β-Glucosidase durchgeführt wird.

Die japanische Offenlegungsschrift 03/085996 offenbart Indophenyl-β-maltooligoside mit 3 bis 8 Glucoseeinheiten, die Indophenylresten als Chromophor und unsubstituierte oder acylsubstituierte OH-Gruppen aufweisen und bei denen die OH-Gruppen in den Positionen $C_4$ und $C_6$ der Glucose am nicht reduzierenden Ende gegebenenfalls mit Alkylidengruppen substituiert sind. Beispielhaft offenbart ist Isopropylidenphenolindophenyl-β-D-maltopentaosid, dessen Bestimmung in Gegenwart der Hilfsenzyme α- und β-Glucosidase erfolgt.

Alle oben genannten Verfahren und Enzymsubstrate für die Bestimmung von α-Amylase weisen jedoch Nachteile auf. So sind die oben genannten Verbindungen mit 5 oder mehr Glucoseeinheiten, bei denen der Substituent am reduzierenden Ende des Zuckers in α- oder β-Stellung verknüpft ist, nicht als direkte Substrate, sondern nur in Anwesenheit von Hilfsenzymen zur Bestimmung der α-Amylase geeignet. Andere Substanzen sind als Substrate ungeeignet, weil sie wenig sensitiv sind und ihre Absorptionsmaxima bei ungünstigen Wellenlängen liegen. So ist beispielsweise bekannt, daß bei Durchführung von α-Amylasetests mit Meßwellenlängen von unterhalb 500 nm Störungen auftreten können, die auf in Körperflüssigkeiten anwesende Verbindungen wie etwa Hämoglobin und Bilirubin zurückzuführen sind.

Eine Aufgabe der vorliegenden Erfindung war es daher, α-Amylasesubstrate bereitzustellen, die im langwelligen Bereich, d.h. oberhalb ca. 550 nm absorbieren und die direkt ohne Erfordernis von Hilfsenzymen von der α-Amylase gespalten werden können.

Die erfindungsgemäße Aufgabe wird gelöst durch Verbindungen der allgemeinen Formel I

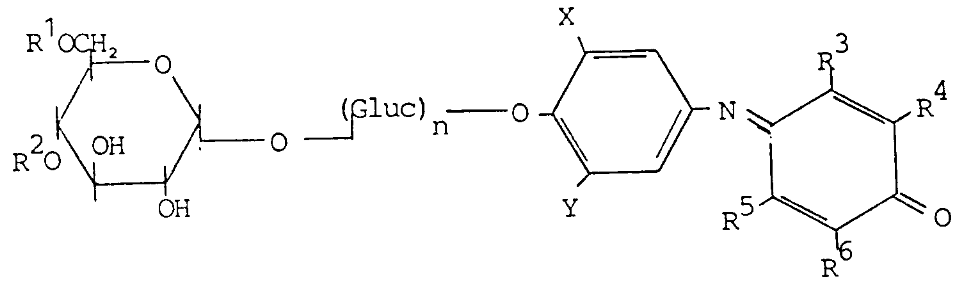

(I)

worin
Gluc ein Glucosemolekül ist und der Indophenolrest in α-Stellung mit dem Zucker verknüpft ist,
n = 1 oder 2,
$R^1$ = H, eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, eine Benzoyl-, Benzyl- oder Phenylgruppe,
$R^2$ = H,
oder worin $R^1$ und $R^2$ zusammen eine Methylenbrücke bilden, deren H-Atome unabhängig voneinander durch je eine Alkylgruppe mit 1 bis 5 C-Atomen oder eine Phenylgruppe substituiert sein können,
$R^3$, $R^4$, $R^5$, $R^6$ = unabhängig voneinander H, eine Halogen-(F, Cl, Br), Nitro-, Acetamid- oder Cyanogruppe, eine Sulfonsäure-, Sulfinsäure- oder Carbonsäuregrupe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, wobei $R^3$ und $R^4$ oder/und $R^5$ und $R^6$ miteinander verbunden sein und kondensierte Ringe bilden können,
X, Y = unabhängig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester-und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe,
unter der Voraussetzung, daß X und Y nicht gleichzeitig H sind.

Die in den obigen Definitionen genannte Arylgruppe ist vorzugsweise Phenyl oder Naphthyl. Die Alkoxygruppe kann eine oder mehrere O-Brücken enthalten, sie kann daher z.B. auch eine Polyethylenglykolkette sein. Die Alkyl-, Cycloalkyl- und Arylreste sind gegebenenfalls substituiert, d.h. sie können Substituenten (wie z.B. Halogengruppen) tragen, die keine chemische Reaktion mit α-Amylase unter den beim enzymatischen Bestimmungstest herrschenden Bedingungen eingehen.

Überraschenderweise sind die Verbindungen der allgemeinen Formel I sehr gut als synthetische Enzymsubstrate für die α-Amylase geeignet, da sie direkt (d.h. in Abwesenheit von Hilfsenzymen) von der α-Amylase gespalten werden können und zugleich eine Absorption im langwelligen Bereich oberhalb ca. 550 nm zeigen. Die Verwendbarkeit der erfindungsgemäßen Substanzen ist umso überraschender, da Verbindungen mit analoger Struktur, bei denen aber an beiden Positionen X und Y des Phenylrings im Indophenolrest Wasserstoffatome vorhanden sind, von der α-Amylase nicht direkt gespalten werden können.

Die Substituenten $R^1$ und $R^2$ am nicht reduzierenden Ende der Verbindung I sind vorzugsweise Wasserstoffatome. Es ist jedoch auch möglich, daß die Verbindung I an diesen Positionen durch Substituenten blockiert ist. Die Einführung solcher Substituenten ist detailliert in der DE-39 29 355 beschrieben. Demgemäß kann die Einführung der Reste $R^1$ und $R^2$ aus den entsprechenden unsubstituierten Verbindungen I erfolgen, indem man diese unter Veretherungsbedingungen mit einer Dialkoxy-Verbindung, vorzugsweise Dialkoxyethan oder dem entsprechenden Benzylderivat umsetzt unter Bildung einer Verbindung der allgemeinen Formel I, in welcher $R^1$ und $R^2$ zusammen eine gegebenenfalls substituierte Methylengruppe bilden.

Es kann auch in das ungeschützte Substrat die gewünschte Substituentengruppe über aktivierte Carbonsäuregruppen wie z.B. über die entsprechenden Orthoester, Säurechloride, Anhydride, aus aktivierten Estern enzymatisch (J.A.C.S 110 (1988), 584-589), aus Acetalen oder direkt aus der Carbonsäure über wasserentziehende Mittel wie z.B. durch die Mitsunobu-Reaktion (Tetrahedron Letters Vol. 30 (1989), 325-326) eingeführt werden. Besonders bevorzugt ist die enzymatische Herstellung, die über die entsprechenden Orthoester als Zwischenprodukte verläuft und die Herstellung über die Mitsunobu-Reaktion. Die Orthoester werden bevorzugt aus den entsprechenden Nitrilen hergestellt (vgl. z.B. Houben-Weyl, Band VI/3

(1965), 300-313). Die Reinigung dieser geschützten Substrate kann z.B. chromatographisch über Ionenaustauscher oder HPLC erfolgen.

In der allgemeinen Formel I bedeutet n = 1 oder 2, daher handelt es sich bei den erfindungsgemäßen Verbindungen um Maltoside oder Maltotrioside. Höhere Maltooligoside werden von der α-Amylase als direkte Substrate nicht mehr akzeptiert. Vorzugsweise handelt es sich bei den erfindungsgemäßen Verbindungen um Maltotrioside.

Die Reste X und Y am Phenylring des Indophenolrests von I können unabhängig voneinander H, eine F-, Cl- oder Br-, Acetamid-, Nitro- oder Cyanogruppe, eine Sulfonsäure-, Sulfinsäure- oder Carbonsäuregruppe sowie entsprechende Säurealkylester-und Säureamidgruppen, eine Alkoxygruppe (mit einer oder mehreren Sauerstoffbrücken, d.h. beispielsweise auch eine Polyethylenglycolkette), eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe sein, unter der Voraussetzung, daß X und Y nicht gleichzeitig H sind. Von den Verbindungen der Formel I sind allgemein diejenigen bevorzugt, bei denen einer der beiden Reste X und Y Wasserstoff und der andere einen von Wasserstoff verschiedenen Substituenten darstellt. Vorzugsweise ist auch mindestens eines von X und Y eine Halogengruppe, besonders bevorzugt ist X eine Halogengruppe und Y Wasserstoff. Am meisten bevorzugt ist es, wenn die Halogengruppe Chlor bedeutet.

Die Reste $R^3$, $R^4$, $R^5$ und $R^6$ in der allgemeinen Formel I stellen die möglichen Substituenten am Benzochinonring des Indophenolrests dar. Dabei können $R^3$, $R^4$, $R^5$ und $R^6$ unabhängigvoneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, wobei $R^3$ und $R^4$ oder/und $R^5$ und $R^6$ miteinander verbunden sein und kondensierte Ringe bilden können, wobei die Alkoxygruppe eine oder mehrere O-Brücken aufweisen kann. Wenn die Reste $R^3$ und $R^4$ oder/und die Reste $R^5$ und $R^6$ miteinander verbunden sind, entstehen kondensierte Ringe. Auch bei einem solchen Substitutionsmuster sind die resultierenden Verbindungen I als direkte α-Amylase-Substrate geeignet.

$R^3$ und $R^5$ bedeuten vorzugsweise Wasserstoff und $R^4$ und $R^6$ bedeuten vorzugsweise Halogen, besonders bevorzugt Chlor. Am meisten bevorzugt von den Verbindungen der allgemeinen Formel I ist 2',2,6-Trichlor-indophenyl-α-maltotriosid, das aufgrund seiner langwelligen Absorption bei einer Meßwellenlänge von 645 nm bestimmt werden kann und hervorragend durch α-Amylase in Abwesenheit von Hilfsenzymen spaltbar ist.

Die Herstellung der erfindungsgemäßen Verbindungen erfolgt auf an sich bekannte Weise, wobei drei verschiedene Verfahren bevorzugt genannt werden können. Bei der ersten und bevorzugten Verfahrensvariante geht man von Maltose oder Maltotriose aus. Diese Verbindungen werden auf an sich bekannte Weise unter Natriumacetat-Katalyse peracetyliert (z.B. durch Umsetzung mit Essigsäureanhydrid), wobei eine Verbindung mit der allgemeinen Formel II erzeugt wird,

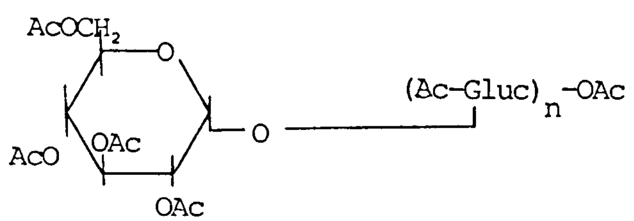

(II)

worin Ac-Gluc eine peracetylierte Glucose bedeutet und der Acetylrest am reduzierenden Ende von II in β-Stellung mit dem Zucker verknüpft ist.

Die Verbindung II wird anschließend in Gegenwart eines Säurekatalysators mit einem substituierten p-Nitrophenol der allgemeinen Formel III umgesetzt

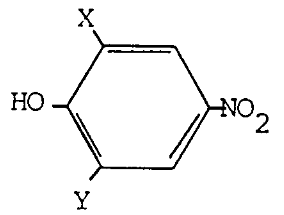

(III)

worin X und Y die oben genannten Bedeutungen besitzen. Als Säurekatalysator für diese Umsetzung sind z.B. Alkyl- oder Arylsulfonsäuren wie etwa p-Toluolsulfonsäure oder vorzugsweise Lewis-Säurekatalysatoren wie etwa $BF_3 \cdot OEt_2$ geeignet. Diese Reaktion ist eine stereospezifische Substitution des Acetylrests am reduzierenden Ende des Zuckers durch das p-Nitrophenol-III, die formal nach einem $S_N2$-Mechanismus verläuft, so daß in der resultierenden Verbindung IV

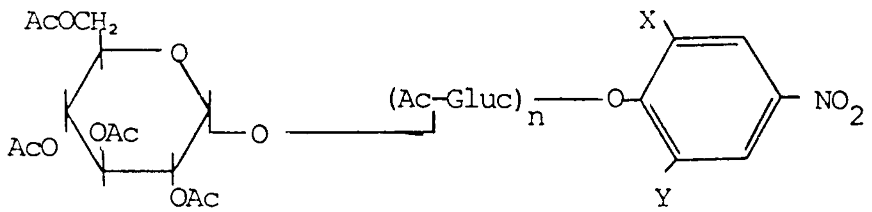

(IV)

der p-Nitrophenylrest in $\alpha$-Stellung mit dem Zucker verknüpft ist. Anschließend wird die Nitrogruppe von IV auf an sich bekannte Weise (z.B. durch katalytische Hydrierung in Gegenwart von Pd-C) zu einer Aminogruppe reduziert. Danach werden die Acetylreste aus der resultierenden Verbindung z.B. durch alkalische Verseifung in Gegenwart von Ammoniak abgespalten. Das deacetylierte Produkt wird schließlich mit einem gegebenenfalls substituierten p-Benzochinon der allgemeinen Formel V umgesetzt,

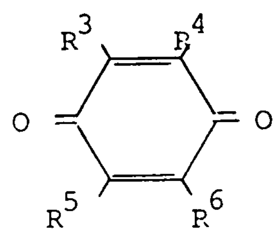

(V)

worin $R^3$, $R^4$, $R^5$ und $R^6$ die oben genannten Bedeutungen besitzen. Auf diese Weise wird eine Verbindung mit der allgemeinen Formel VI erzeugt,

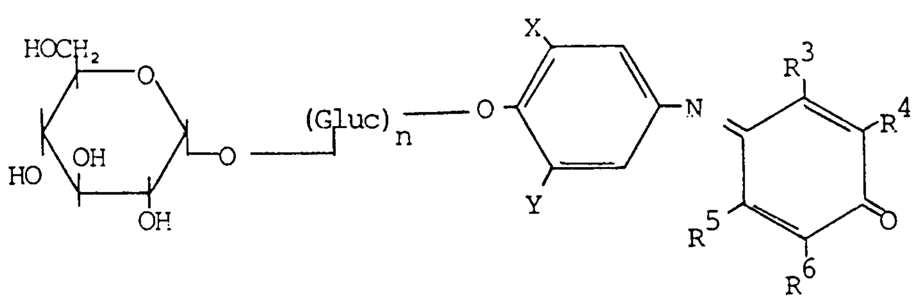

(VI)

worin der Indophenolrest in $\alpha$-Stellung mit dem Zucker verknüpft ist. Diese Umsetzung findet vorzugsweise in einem wasserfreien aprotischen Lösungsmittel, wie etwa Dimethylformamid oder Dioxan oder einem Gemisch davon, in Gegenwart von Trifluoressigsäure statt.

Gegebenenfalls kann in einem weiteren Schritt eine Substitution der Wasserstoffatome an den Positionen 4 und 6 des Glucoserests am nicht reduzierenden Ende des Zuckers mit den Resten $R^1$ und $R^2$

erfolgen, wie bereits oben ausführlich erläutert wurde.

Alternativ dazu kann die Herstellung der Verbindung der allgemeinen Formel I dadurch erfolgen, daß man eine peracetylierte $\beta$-Maltose oder $\beta$-Maltotriose mit einem Indophenol der allgemeinen Formel VII umsetzt,

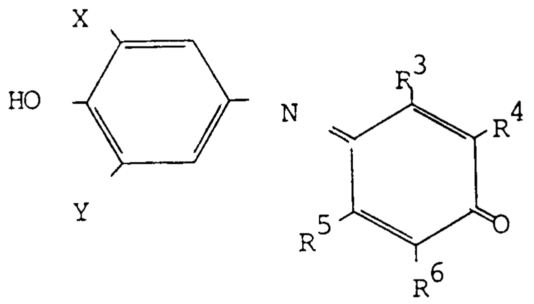

(VII)

worin X, Y, $R^3$, $R^4$, $R^5$ und $R^6$ die oben genannten Bedeutungen besitzen. Die weitere Aufarbeitung des resultierenden Produkts, d.h. Deacetylierung und gegebenenfalls Derivatisierung am nicht reduzierenden Ende, erfolgt analog der ersten Verfahrensvariante.

Eine dritte Möglichkeit zur Herstellung der erfindungsgemäßen Verbindungen ist es, wenn man nach einer der oben beschriebenen Verfahrensweisen zunächst ein Indophenyl-$\alpha$-glucosid-Derivat der Formel VIII herstellt,

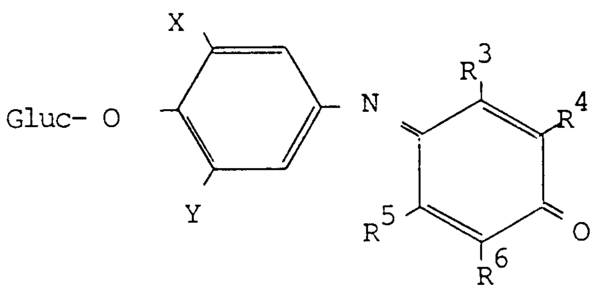

(VIII)

worin die jeweiligen Reste die oben genannten Bedeutungen besitzen und diese Verbindung anschließend in Gegenwart von Cyclodextrin, Amylose oder Stärke in Gegenwart von Cyclodextringlucosyltransferase durch "trimming" zu den erfindungsgemäßen Maltosid- und Maltotriosid-Derivaten umsetzt.

Verfahren zur Herstellung von $\beta$-Maltooligosidderivaten, die sich von den erfindungsgemäßen Verbindungen durch die Stellung des Substituenten am reduzierenden Ende des Zuckers unterscheiden, sind in der japanischen Offenlegungsschrift 64-42497 detailliert für die entsprechenden Indophenyl-$\beta$-Glucosidderivate offenbart. Die Herstellung der erfindungsgemäßen $\alpha$-Maltooligosidderivate unterscheidet sich prinzipiell nur dadurch, daß man von einem peracetylierten $\beta$-Maltooligosidderivat ausgeht, während man zur Herstellung der $\beta$-Verbindungen von einem peracetylierten $\alpha$-Maltooligosidderivat ausgeht.

Die Herstellung von $\alpha$-Maltooligosidderivaten, die sich von den erfindungsgemäßen Substanzen durch die Art des Substituenten unterscheiden, ist in der EP-A 0 263 435 offenbart.

Ein weiterer Gegenstand der vorliegenden Erfindung ist ein Verfahren zur Bestimmung der Aktivität von $\alpha$-Amylase in wäßriger Lösung, insbesondere in Körperflüssigkeiten mit einem synthetischen Enzymsubstrat, wobei man als Enzymsubstrat eine Verbindung mit der allgemeinen Formel I nach Anspruch 1 verwendet. Vorzugsweise verwendet man bei diesem Verfahren eine Verbindung I, in der $R^1$ und $R^2$ Wasserstoff bedeuten oder zusammen eine Methylenbrücke bilden, deren H-Atome unabhängig voneinander durch je eine Alkylgruppe mit 1 bis 5 C-Atomen oder eine Phenylgruppe substituiert sein können. Weiterhin ist bevorzugt, daß einer der Substituenten X und Y am Phenylring des Indophenolrests Wasserstoff ist und der andere Substituent von Wasserstoff verschieden ist. Weiterhin ist bevorzugt, wenn mindestens einer der Reste X und Y eine Halogengruppe bedeutet. Besonders bevorzugt ist die Verwendung von 2',2,6-Trichlor-indophenyl-$\alpha$-maltotriosid als Enzymsubstrat.

Mit dem erfindungsgemäßen Verfahren läßt sich eine direkte Aktivitätsbestimmung von $\alpha$-Amylase, d.h. eine Aktivitätsbestimmung in Abwesenheit von Hilfsenzymen wie etwa $\alpha$-Glucosidase oder $\beta$-Glucosidase durchführen.

Ferner ist es bei dem erfindungsgemäßen Verfahren möglich, spezifisch die Aktivität von Pankreas-α-Amylase in Gegenwart von Speichel-α-Amylase unter Verwendung eines spezifischen Hemmstoffsystems für Speichel-α-Amylase durchführt. Ein solches Hemmstoffsystem, das einen oder mehrere monoklonale Antikörper umfaßt, wird z.B. in der DE 39 29 355 offenbart. Geeignete Antikörper sind z.B. in der EP-A 0 191 284, der EP-A 0 150 309 und in der EP-A 0 209 154 offenbart. Durch Kombination des erfindungsgemäßen Enzymsubstrats und des Hemmstoffs erfolgt die Bestimmung der Pankreas-α-Amylase sehr selektiv und sehr genau.

Weiterhin betrifft die vorliegende Erfindung ein Reagenz zur direkten spezifischen Bestimmung von α-Amylase mit einem synthetischen Enyzmsubstrat, das als Enzymsubstrat eine Verbindung der allgemeinen Formel I enthält. Vorzugsweise enthält das erfindungsgemäße Reagenz als Enzymsubstrat eine Verbindung I, in der $R^1$ und $R^2$ Wasserstoff bedeuten oder zusammen eine Methylenbrücke, deren H-Atome unabhängig voneinander durch je eine Alkylgruppe mit 1 bis 5 C-Atomen oder eine Phenylgruppe substituiert sein können. Weiterhin ist bevorzugt, daß in dem verwendeten Substrat der Formel I einer der Substituenten X und Y Wasserstoff ist und der andere Substituent von Wasserstoff verschieden ist. Weiterhin ist bevorzugt, wenn mindestens einer der Reste X und Y eine Halogengruppe bedeutet. Besonders bevorzugt ist ein erfindungsgemäßes Reagenz, das als Substrat 2',2,6-Trichlor-indophenyl-α-maltotriosid enthält. Die Endkonzentration des erfindungsgemäßen Substrats in der Enzymtestlösung beträgt vorzugsweise 1 bis 10 mmol/l, besonders bevorzugt 3 bis 7 mmol/l. Das erfindungsgemäße Reagenz kann auch zusätzlich einen Aktivator für die α-Amylase enthalten, z.B. ein Azid- oder Rhodanidsalz. Der Aktivator wird vorzugsweise mit einer Endkonzentration von 50 bis 700 mmol, besonders bevorzugt von 100 bis 500 mmol/l eingesetzt. Weiterhin kann das erfindungsgemäße Reagenz auch ein spezifisches Hemmstoffsystem für Speichel-α-Amylase enthalten, das aus einem oder mehreren monoklonalen Antikörpern besteht. Auf diese Weise ist eine selektive Bestimmung von Pankreas-α-Amylase möglich.

Die Erfindung betrifft weiterhin die Verwendung einer Verbindung der allgemeinen Formel I als synthetisches Enzymsubstrat, insbesondere zur direkten Bestimmung der enzymatischen Aktivität von α-Amylase.

Die Erfindung wird weiter durch die folgenden Beispiele in Verbindung mit der Zeichnung erläutert.

In der Zeichnung zeigt Fig. 1 die chemischen Strukturformeln der in Beispiel 2 getesteten α-Amylasesubstrate.

## Beispiel 1

Synthese von 2',2,6-Trichlor-indophenyl-α-maltotriosid

**a) Deca-acetyl-(2-chlor-4-nitrophenyl)-α-maltotriosid**

0,1 mol peracetylierte β-Maltotriose, 0,3 mol 2-Chlor-4-nitrophenol und 25 ml $BF_3 \cdot OEt_2$ werden in einem Gemisch von 450 ml wasserfreiem Toluol und 100 ml wasserfreiem Dichlorethan 32 Stunden bei 45 °C unter Feuchtigkeitsausschluß gerührt. Anschließend wird 500 ml Dichlormethan zugegeben und mit 800 ml gesättigter $Na_2CO_3$-Lösung ausgeschüttelt. Die organische Phase wird nach Trocknung über $MgSO_4$ eingeengt. Der Rückstand wird über Kieselgel chromatographiert (Laufmittel: Essigester/Hexan 2:1).
Ausbeute: 27 g (25 %)
DC: $R_f$ = 0,59 (Kieselgel; Toluol/Aceton 7/4)

**b) Deca-acetyl-(2-chlor-4-aminophenyl)-α-maltotriosid**

6,5 g (6 mmol) des Produktes aus 1a) werden in einem Gemisch von 160 ml Dioxan, 60 ml Methanol, 2 ml Eisessig gelöst und mit 0,65 g 10 %igem Pd-C versetzt. Die Hydrierung wird 7 Stunden unter einem Druck von 0,9 bar durchgeführt. Nach Abfiltrieren des Katalysators und Entfernen des Lösungsmittels wird der Rückstand über Kieselgelchromatographie (Laufmittel: Toluol/Aceton 7:3) gereinigt.
Ausbeute: 1,6 g (25 %)
DC: $R_f$ = 0,42 (Kieselgel; Toluol/Aceton 7/4)

**c) 2-Chlor-4-aminophenyl-α-maltotriosid**

1 g (1 mmol) des Produkts aus 1b) werden in 50 ml Methanol, 5 ml Wasser und 10 ml 25 % Ammoniak versetzt und 20 Stunden bei Raumtemperatur gerührt. Das Lösungsmittel wird abgezogen und das Produkt ohne weitere Aufreinigung weiter umgesetzt.
Ausbeute: 680 mg
DC: $R_f$ = 0,47 (Kieselgel; Butanol/Eisessig/Wasser 10/3/5)

**d) 2',2,6-Trichlor-indophenyl-α-maltotriosid**

680 mg des Produkts aus 1c) werden in 10 ml wasserfreiem Dimethylformamid, 30 ml wasserfreiem Dioxan, 0,2 ml Trifluoressigsäure gelöst und mit 1 g (5,8 mmol) 2,6-Dichlor-benzochinon und 3

Spatelspitzen Molekularsieb versetzt. Das Gemisch wird 2 Stunden unter Feuchtigkeitsausschluß bei Raumtemperatur gerührt. Anschließend wird filtriert, eingeengt, der Rückstand mit Wasser aufgenommen und nochmals filtriert. Das Filtrat wird über eine Gelchromatographie (LH 20, Laufmittel: Wasser) vorbehandelt. Abschließend wird über präparative HPLC endgereinigt (RP 18; Gradient: Wasser/Isopropanol; 0,1 % Trifluoressigsäure: 80/20 % → 65/35 %).
Ausbeute: 60 mg (8 %)
DC: $R_f$ = 0,62 (Kieselgel; Butanol/Eisessig/Wasser 10/3/5)

Beispiel 2

Verwendung von 2',2,6-Trichlor-indophenyl-α-maltotriosid als Substrat zur direkten α-Amylase-Bestimmung

Es wurde mit den in der folgenden Tabelle und in Fig. 1 aufgeführten Substraten, die Amylaseaktivität von Human-Pankreas-α-Amylase (HPA) und Human-Speichel-α-Amylase (HSA) untersucht. Die folgenden Reagenzien wurden verwendet:

Reagenz 1:

| | | |
|---|---|---|
| 4-Morpholinethansulfonat | | |
| (=MES)-Puffer | | 61,6 mMol/l pH 6,0 |
| enthaltend | NaCl | 57,1 mMol/l |
| | Calciumacetat | 5,6 mMol/l |

Reagenz 2:

Substrat (wie in der Tabelle angegeben) 49,3 mMol/l

Reagenz 1 enthält gegebenenfalls noch Aktivator, wie in Tabelle angegeben, sowie - als Hemmstoffe für die Speichel-α-Amylase- von den Zellinien NCACC 84122003 (MAK I) und NCACC 84111301 (MAK II) produzierte Antikörper. Zur Durchführung der Bestimmung wird 1,00 ml Reagenz 1 mit 0,02 ml der Probe gemischt und auf 37 °C temperiert. Anschließend wird 0,10 ml Reagenz 2 zugegeben, gemischt und nach 1,2 und 3 Minuten die Extinktion bei der jeweiligen Meßwellenlänge (s. Tabelle) abgelesen. Es wird der Mittelwert der Extinktionsdifferenz/Minute gebildet.

Berechnung:

$$\text{U/L Probe} = \frac{\Delta E/\text{min} \times 1{,}12 \times 1000}{\epsilon \times 0{,}02}$$

Die Endkonzentrationen im Testansatz betragen:

| | |
|---|---|
| MES-Puffer, pH 6,0 | 55 mmol/l |
| NaCl | 51 mmol/l |
| Calciumacetat | 5 mmol/l |
| Substrat gegebenenfalls Aktivator | 4,44 mmol/l |
| Natriumazid | 152 mmol/l |
| bzw. Kaliumthiocyanat | 400 mmol/l |
| MAK I | ca. 5 bis 8 mg/l |
| MAK II | ca. 2 bis 3 mg/l |

Als Probe wird Humanpankreas-Amylase (HPA) verwendet. Die Messung erfolgt bei 37 °C bei der jeweils angegebenen Meßwellenlänge.
Die Ergebnisse sind der Tabelle zu entnehmen.

Tabelle

| Substrat | Meßwellenlänge [nm] | 400 mMol/l KSCN HPA [ΔE/min] |
|---|---|---|
| 2',2,6-Trichlor-indophenyl-α-maltotriosid | 645 | 0,063 |
| 2,6-Dimethyl-indophenylα-maltotriosid | 578 | 0* |
| 2,6-Dichlor-indophenylα-maltotriosid | 578 | 0 |

* Mit 150 mmol/l $NaN_3$ anstatt KSCN

Die erhaltenen Ergebnisse zeigen, daß nur Indophenylderivate, die an Position 2' des Phenylringes einen von Wasserstoff verschiedenen Substituenten, insbesondere einen Halogensubstituenten besitzen, direkte Amylase-Substrate darstellen. Fehlt der Substituent in der 2-Position, tritt keine Amylase-Spaltung ein.

Beispiel 3

**a) Isopropyliden-2-chlor-4-nitrophenyl-α-D-maltotriosid**
14,25 g Deca-acetyl-(2-chlor-4-nitrophenyl)-α-maltotriosid
aus Beispiel 1a) werden in 450 ml Methanol gelöst. Dazu gibt man 45 ml Wasser und 90 ml konz. wäßrige Ammoniaklösung und rührt 16 Stunden bei Raumtemperatur. Man engt ein und löst 660 mg (1 mmol) des erhaltenen 2-Cl-4-Nitrophenyl-α-D-maltotriosids in 15 ml DMF. Nacheinander werden 1,25 ml (10 mmol) 2,2-Dimethoxypropan (Merck 802936) und 172 mg (1 mmol) p-Toluolsulfonsäure, wasserfrei, zugegeben. Bei 48 °C läßt man unter Rühren 80 Min reagieren. Dann wird der Ansatz in 15 ml 0,24 mol/l $NaHCO_3$-Lsg getropft. Das Gemisch wird 2 mal mit je 40 ml Essigsäureethylester geschüttelt. Die Wasserphase wird eingeengt und anschließend auf einer Sephadex LH20-Säule (Pharmacia) chromatographiert. Eluent: Wasser.
Die geeigneten Fraktionen werden lyophilisiert. Ausbeute: 150 mg
DC: Rf = 0,51 (Kieselgel-DC, Merck 5735,
Fließmittel 1-Butanol/Eisessig/Wasser = 10/3/5)
**b) Isopropyliden-2-chlor-4-aminophenyl-α-D-maltotriosid**
150 mg Isopropyliden-2-chlor-4-nitrophenyl-α-D-maltotriosid
aus Beispiel 3a) werden in 10 ml Methanol gelöst und mit 25 ml Tetrahydrofuran und 30 mg Pd auf Aktivkohle (10 % Pd, Merck 807104) versetzt.
Man hydriert 2 Stunden unter heftigem Schütteln ohne Überdruck. Der gesamte Ansatz wird vom Katalysator filtriert, eingedampft und nach 30 Min. an der Hochvakuumpumpe getrocknet.
Ausbeute: 140 mg
DC: Rf = 0,44 (Kieselgel, Merck 5735.
Fließmittel 1-Butanol/Eisessig/Wasser = 10/3/5)
**c) Isopropyliden-2',2,6-trichlorindophenyl-α-D-maltotriosid**
140 mg (0,2 mmol) Isopropyliden-2-chlor-4-aminophenyl-α-D-maltotriosid aus Beispiel 3b) werden in 1,5 ml Dimethylformamid, destilliert und wasserfrei, gelöst. Dazu werden 3 ml Dioxan, wasserfrei, und 3 Tropfen $BF_3$-Ethyletherkomplex gegeben. Man kühlt auf 10 °C ab und gibt 0,22 g (1,2 mmol) 2,6-Dichlorbenzochinon zu. Man rührt 15 Min. bei 10 °C, läßt dann auf Raumtemperatur erwärmen und rührt noch weitere 60 Min.
Zu dem Reaktionsansatz werden 40 ml Diethylether gegeben, worauf das Produkt ausflockt. Man filtriert ab und wäscht den Rückstand mit Diethylether. Das Produkt wird durch Aufreinigung an Sephadex LH 20 (Pharmacia) mit Wasser als Eluent erhalten.
DC: Rf = 0,60 (Kieselgel, Merck 5735,
Fließmittel 1-Butanol/ Eisessig/Wasser = 10/3/5)

Beispiel 4

Synthese von 2'-Chlor-2-methyl-indophenyl-α-maltotriosid

3,5 g des Produkts aus 1c) werden in 15 ml wasserfreiem Dimethylformamid, 60 ml wasserfreiem Dioxan, 1,3 ml $BF_3$-Ethyletherkomplex gelöst und mit 2,45 g (20 mmol) 2-Methyl-4-benzochinon und 1 Spatellöffel Molekularsieb versetzt. Das Gemisch wird über Nacht (16 Std.) bei Raumtemperatur und unter Feuchtigkeitsausschluß gerührt. Anschließend werden 200 ml Essigsäureethylester zugegeben und der hieraus entstandene Niederschlag über eine G4-Fritte abgesaugt. Der Rückstand wird in Wasser gelöst und über eine Sephadex LH 20-Säule gereinigt.
Ausbeute: 230 mg
DC: Rf = 0,44 (Kieselgel;
1-Butanol/Eisessig/Wasser 10/3/5)

Beispiel 5

Synthese von 2'-Chlor-2,6-dimethyl-indophenyl-α-maltotriosid

500 mg des Produkts aus 1c) werden in 5 ml wasserfreiem Dimethylformamid, 10 ml wasserfreiem Dioxan, 0,2 ml $BF_3$-Ethyletherkomplex gelöst und mit 0,55 g ( 3 mmol) 2,6-Dimethyl-4-benzochinon und 2 Spatelspitzen Molekularsieb versetzt. Das Gemisch wird über Nacht bei Raumtemperatur und unter Feuchtigkeitsausschluß gerührt. Anschließend werden 20 ml Essigsäureethylester und 20 ml Diethylether zugegeben und der entstandene Niederschlag über eine G4-Fritte abgesaugt. Dieser orange-rote Rückstand wird in Wasser gelöst und über eine Sephadex LH 20-Säule gereinigt.
Ausbeute: 35 mg
DC: Rf = 0,44 (Kieselgel;
1-Butanol/Eisessig/Wasser 10/3/5)

Beispiel 6

Verwendung der gemäß Beispiel 4 und Beispiel 5 hergestellten Substanzen als Substrate zur direkten α-Amylase-Bestimmung.

Es wird analog zu Beispiel 2 vorgegangen.

Die Ergebnisse sind der Tabelle 2 zu entnehmen.

Tabelle 2

| Substrat | Meßwellen länge [nm] | 400 mmol/l KSCN HPA relativer Wert [ΔE/min] |
|---|---|---|
| 2',2,6-Trichlor-indo phenyl-α-maltotriosid (Beispiel 1) | 645 | 1 |
| 2'-Chlor-2-methylindo phenyl-α-maltotriosid (Beispiel 4) | 595 | 0,35* |
| 2'-Chlor-2,6-dimethyl indophenyl-α-maltotriosid (Beispiel 5) | 573 | 0,27* |

* bei pH 8,0 gemessen

## Patentansprüche

**1.** Verbindung der allgemeinen Formel (I)

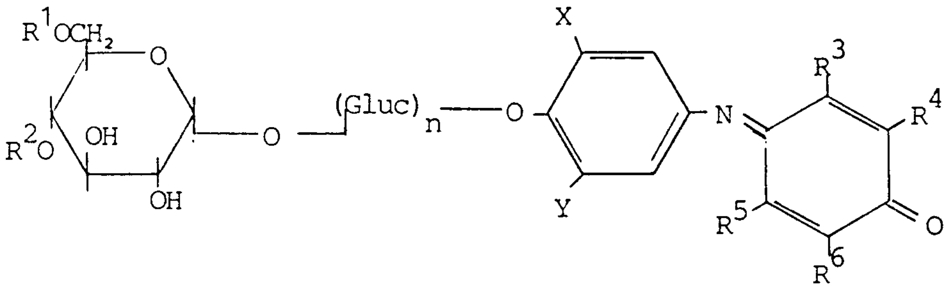

(I)

worin
Gluc ein Glucosemolekül ist und der Indophenolrest in $\alpha$-Stellung mit dem Zucker verknüpft ist,
n = 1 oder 2,
$R^1$ = H, eine geradkettige oder verzweigte Alkyl- oder Alkoylgruppe mit 1 bis 6 C-Atomen, eine Cycloalkyl- oder Cycloalkoylgruppe mit 3 bis 6 C-Atomen, eine Benzoyl-, Benzyl- oder Phenylgruppe,
$R^2$ = H,
oder worin $R^1$ und $R^2$ zusammen eine Methylenbrücke bilden, deren H-Atome unabhängig voneinander durch je eine Alkylgruppe mit 1 bis 5 C-Atomen oder eine Phenylgruppe substituiert sein können,
$R^3$, $R^4$, $R^5$, $R^6$ = unabhängig voneinander H, eine Halogen-(F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe, wobei $R^3$ und $R^4$ oder/und $R^5$ und $R^6$ miteinander verbunden sein und kondensierte Ringe bilden können,
X, Y = unabhängig voneinander H, eine Halogen- (F, Cl, Br), Nitro-, Acetamid- oder Cyanogruppe, eine Sulfonsäure-, Sulfinsäure- oder Carbonsäuregruppe sowie entsprechende Säurealkylester- und Säureamidgruppen, eine Alkoxygruppe, eine geradkettige oder verzweigte, gegebenenfalls substituierte Alkylgruppe mit 1 bis 6 C-Atomen, eine gegebenenfalls substituierte Cycloalkylgruppe mit 3 bis 6 C-Atomen oder eine gegebenenfalls substituierte Arylgruppe,
unter der Voraussetzung, daß X und Y nicht gleichzeitig H sind.

**2.** Verbindung nach Anspruch 1,
**dadurch gekennzeichnet,**
daß $R^1$ und $R^2$ Wasserstoff bedeuten.

**3.** Verbindung nach Anspruch 1 oder 2,
**dadurch gekennzeichnet,**
daß n = 2 bedeutet.

**4.** Verbindung nach einem der Ansprüche 1 bis 3,
**dadurch gekennzeichnet,**
daß einer der beiden Reste X und Y Wasserstoff und der andere von Wasserstoff verschieden ist.

**5.** Verbindung nach einem der Ansprüche 1 bis 4,
**dadurch gekennzeichnet,**
daß mindestens einer der Reste X und Y eine Halogengruppe bedeutet.

**6.** Verbindung nach Anspruch 5,
**dadurch gekennzeichnet,**
daß die Halogengruppe ein Chlor ist.

**7.** Verbindung nach einem der Ansprüche 1 bis 6,
**dadurch gekennzeichnet,**
daß $R^3$ und $R^5$ Wasserstoff bedeuten und $R^4$ und $R^6$ Halogen bedeuten.

**8.** Verbindung nach Anspruch 7,
**dadurch gekennzeichnet,**

daß $R^4$ und $R^6$ Chlor bedeuten.

**9.** 2',2,6-Trichlor-indophenyl-α-maltotriosid.

**10.** Verfahren zur Herstellung einer Verbindung mit der allgemeinen Formel I nach Anspruch 1,
**dadurch gekennzeichnet,**
daß man
(a) Maltose oder Maltotriose auf an sich bekannte Weise peracetyliert, wobei eine Verbindung mit der allgemeinen Formel II erzeugt wird

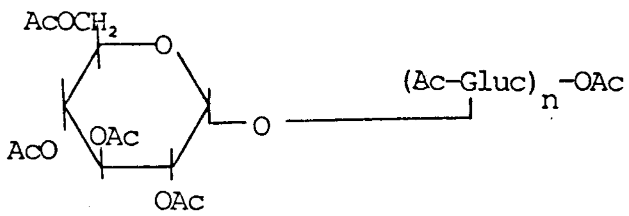

(II)

worin Ac-Gluc eine peracetylierte Glucose bedeutet und der Acetylrest am reduzierenden Ende von II in β-Stellung mit dem Zucker verknüpft ist,
(b) die Verbindung II in Gegenwart eines Säure-Katalysators mit einem substituierten p-Nitrophenol der allgemeinen Formel III umsetzt

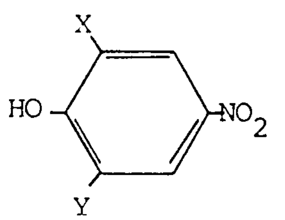

(III)

worin X und Y die in Anspruch 1 genannten Bedeutungen besitzen,
wodurch eine Verbindung mit der allgemeinen Formel IV erzeugt wird,

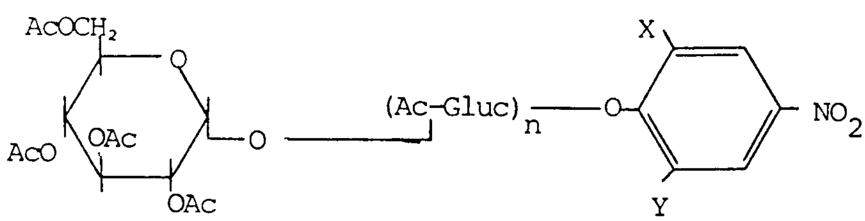

(IV)

worin der p-Nitrophenylrest in α-Stellung mit dem Zucker verknüpft ist,
(c) die Nitrogruppe von IV auf an sich bekannte Weise zu einer Aminogruppe reduziert,
(d) die Acetylreste der aus Schritt (c) resultierenden Verbindung abspaltet,
(e) die aus Schritt (d) resultierende Verbindung mit einem gegebenenfalls substituierten p-Benzochinon der allgemeinen Formel V umsetzt,

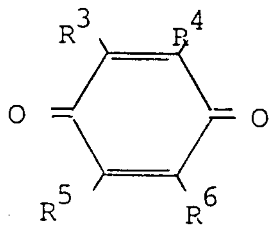

(V)

worin $R^3$, $R^4$, $R^5$ und $R^6$ die in Anspruch 1 genannten Bedeutungen besitzen, wobei eine Verbindung mit der allgemeinen Formel VI erzeugt wird,

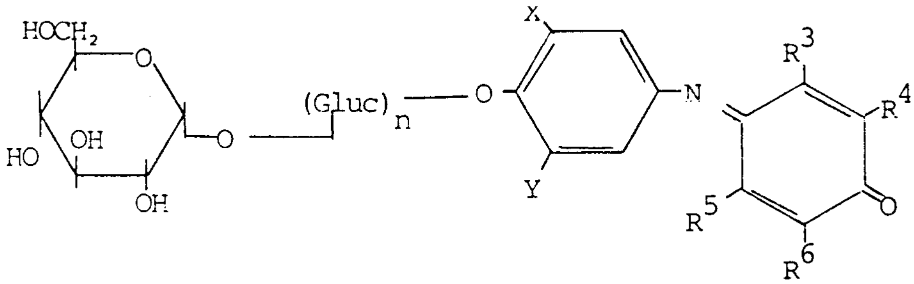

(VI)

worin der Indophenylrest in $\alpha$-Stellung mit dem Zucker verknüpft ist, und gegebenenfalls

(f) auf an sich bekannte Weise eine Substitution der Positionen 4 und 6 am nicht reduzierenden Ende des Maltose- bzw. Maltotrioserests mit den Resten $R^1$ und $R^2$ einführt, wobei eine Verbindung mit der allgemeinen Formel I erzeugt wird,

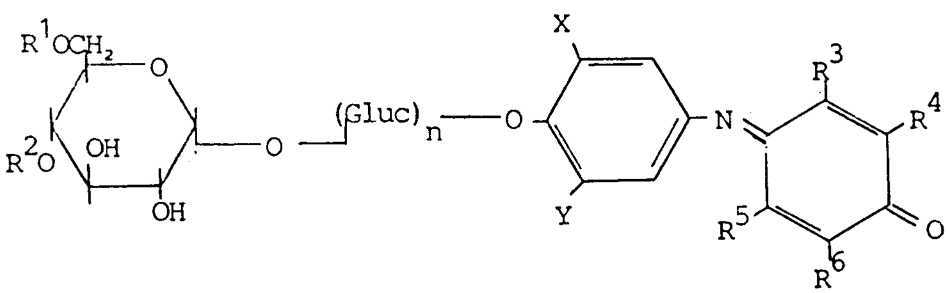

(I)

worin alle Symbole die in Anspruch 1 genannten Bedeutungen besitzen.

**11.** Verfahren zur Bestimmung der Aktivität von $\alpha$-Amylase in wäßriger Lösung, insbesondere in Körperflüssigkeiten mit einem synthetischen Enzymsubstrat,
**dadurch gekennzeichnet,**
daß man als Enzymsubstrat eine Verbindung mit der allgemeinen Formel I nach Anspruch 1 verwendet.

**12.** Verfahren nach Anspruch 11,
**dadurch gekennzeichnet,**
daß man eine Verbindung I verwendet, in der $R^1$ und $R^2$ Wasserstoff bedeuten.

**13.** Verfahren nach Anspruch 11 oder 12,
**dadurch gekennzeichnet,**
daß man eine Verbindung I verwendet, in der einer der beiden Reste X und Y Wasserstoff und der andere von Wasserstoff verschieden ist.

**14.** Verfahren nach Anspruch 13,
**dadurch gekennzeichnet,**
daß man 2',2,6-Trichlor-indophenyl-$\alpha$-maltotriosid als Enzymsubstrat verwendet.

15. Verfahren nach einem der Ansprüche 11 bis 14,
**dadurch gekennzeichnet,**
daß man die Aktivitätsbestimmung von α-Amylase in Abwesenheit von Hilfsenzymen durchführt.

16. Verfahren nach einem der Ansprüche 11 bis 15,
**dadurch gekennzeichnet,**
daß man spezifisch die Aktivität von Pankreas-α-Amylase in Gegenwart von Speichel-α-Amylase unter Verwendung eines spezifischen Hemmstoff-Systems für Speichel-α-Amylase bestimmt.

17. Verfahren nach Anspruch 16,
**dadurch gekennzeichnet,**
daß man als Hemmstoffsystem einen oder mehrere monoklonale Antikörper verwendet.

18. Reagenz zur direkten spezifischen Bestimmung von α-Amylase mit einem synthetischen Enzymsubstrat,
**dadurch gekennzeichnet,**
daß es als Enzymsubstrat eine Verbindung der allgemeinen Formel I nach Anspruch 1 enthält.

19. Reagenz nach Anspruch 18,
**dadurch gekennzeichnet,**
daß es als Substrat eine Verbindung I enthält, in der $R^1$ und $R^2$ Wasserstoff bedeuten.

20. Reagenz nach Anspruch 18 oder 19,
**dadurch gekennzeichnet,**
daß es als Substrat eine Verbindung I enthält, in der einer der beiden Reste X und Y Wasserstoff und der andere von Wasserstoff verschieden ist.

21. Reagenz nach Anspruch 20,
**dadurch gekennzeichnet,**
daß es als Substrat 2',2,6-Trichlor-indophenyl-α-maltotriosid enthält.

22. Reagenz nach einem der Ansprüche 18 bis 21,
**dadurch gekennzeichnet,**
daß es zusätzlich einen Aktivator enthält.

23. Reagenz nach Anspruch 22,
**dadurch gekennzeichnet,**
daß der Aktivator ein Azid- oder ein Rhodanidsalz ist.

24. Reagenz nach einem der Ansprüche 18 bis 23,
**dadurch gekennzeichnet,**
daß es ein spezifisches Hemmstoff-System für Speichel-α-Amylase enthält.

25. Reagenz nach Anspruch 24,
**dadurch gekennzeichnet,**
daß das Hemmstoff-System aus einem oder mehreren monoklonalen Antikörpern besteht.

26. Verwendung einer Verbindung nach einem der Ansprüche 1 bis 9 als synthetisches Enzymsubstrat.

27. Verwendung nach Anspruch 26 zur direkten Bestimmung der enzymatischen Aktivität von α-Amylase.

Fig. 1

2',2,6-Trichlorindophenyl-
alpha-maltotriosid :

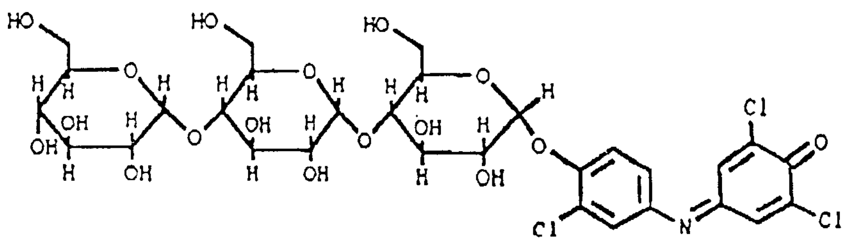

2,6-Dichlorindophenyl-
alpha-maltotriosid:

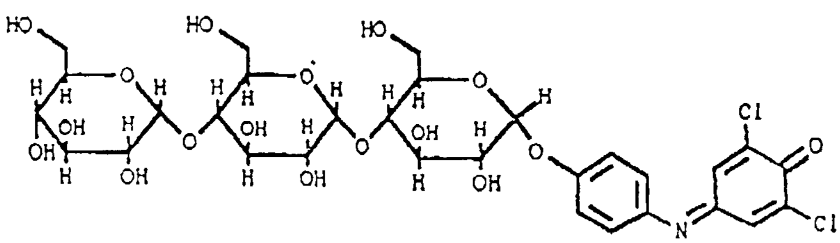

2,6-Dimethylindophenyl-
alpha-maltotriosid:

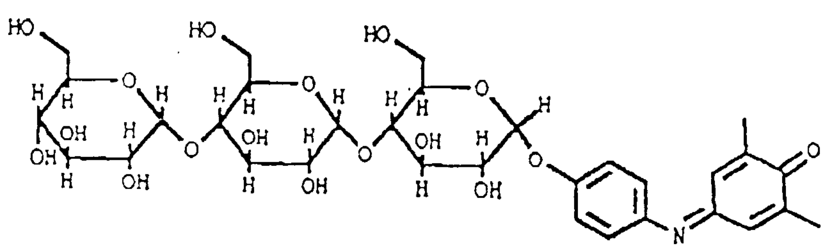

Europäisches Patentamt

# EUROPÄISCHER RECHERCHENBERICHT

Nummer der Anmeldung

EP 92 10 6895

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y<br><br><br><br><br><br><br><br><br>D | CHEMICAL ABSTRACTS, vol. 114, 1991, Columbus, Ohio, US; abstract no. 185925U, SHINOKI ET AL: 'Preparation of Indophenol alpha-Glycosides as Reagents for alpha-Amylase Analysis' Seite 836 ; * Zusammenfassung *<br>& JP-A-2 306 990 (FUJI)<br>--- | 1-27 | C07H15/203<br>C12Q1/40 |
| D,Y | EP-A-0 263 435 (HOECHST CELANESE CORP) * Seite 13, Zeile 30 - Seite 14, Zeile 21; Ansprüche 1-52 *<br>--- | 1-27 | |
| A | CHEMICAL AND PHARMACEUTICAL BULLETIN Bd. 38, Nr. 1, 1990, Seiten 13 - 18; S. TOKUTAKE ET AL.: 'Glycosides Having Chromophores as Substrates for Sensitive Enzyme Analysis. 1.' * das ganze Dokument *<br>----- | 1-27 | RECHERCHIERTE SACHGEBIETE (Int. Cl.5 )<br>C07H<br>C12Q |

Der vorliegende Recherchenbericht wurde für alle Patentansprüche erstellt

| Recherchenort | Abschlußdatum der Recherche | Prüfer |
|---|---|---|
| DEN HAAG | 14 AUGUST 1992 | BRENNAN J. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument